# NEUE EUROPÄISCHE PATENTSCHRIFT

(11) **EP 0 626 361 B2**
(45) Veröffentlichungstag und Bekanntmachung des Hinweises auf die Entscheidung über den Einspruch: **12.07.2000**
(45) Hinweis auf die Patenterteilung: 05.02.1997
(21) Anmeldenummer: 94107473.4
(22) Anmeldetag: 13.05.1994
(51) Int. Cl.: C07B 41/04, C07B 45/06, C07C 205/37, C07C 49/84, C07C 201/12, C07C 45/71

(54) **Verfahren zur Herstellung von Difluormethoxy- und Difluormethylthioarenen**
Process for the preparation of difluoromethoxy- and difluoromethylthioarenes
Procédé pour la préparation de difluorométhoxy- et difluorométhylthioarènes

(30) Priorität: 25.05.1993 DE 4317322
(43) Veröffentlichungstag der Anmeldung: 30.11.1994
(73) Patentinhaber: BAYER AG, 51368 Leverkusen (DE)
(72) Erfinder: Müller, Klaus-Helmut, Dr., D-40593 Düsseldorf (DE)

(56) Entgegenhaltungen:
- EP-A- 0 101 570
- JP-A2- 4 356 439
- JOURNAL OF FLUORINE CHEMISTRY, Bd.41, 1988 Seiten 247 - 261 B. R. LANGLOIS 'IMPROVEMENT IN THE SYNTHESIS OF ARYL DIFLUOROMETHYL ETHERS AND THIOETHERS BY USING A SOLID-LIQUID PHASE-TRANSFER TECHNIQUE'
- CHEMICAL ABSTRACTS, vol. 115, no. 25, 23. Dezember 1991, Columbus, Ohio, US; abstract no. 279585f, I LACKO ET AL 'PREPARATION OF ALKYL PHENYL SULFIDES BY PHASE-TRANSFER THIOETHERIFICATION OF SODIUM THIOPHENOLATE' Seite 968 ;Spalte 1 ; & CS-A-266 277
- CHEMICAL ABSTRACTS, vol. 103, no. 13, 30. September 1985, Columbus, Ohio, US; abstract no. 104979, '1-(3-NITROPHENYL)-3- METHYL-4-DIFLUOROMETHYL-delta-2-1,2,4-TRIA ZOLIN-5-ONE' Seite 625 ;Spalte 1 ; & JP-A-60 048 975 (NIHON NOHYAKU)
- Landini D., Maia A., Rampoldi A., J. Org. Chem. 1986, p. 3187-3191

## Beschreibung

Die Erfindung betrifft ein neues Verfahren zur Herstellung von Difluormethoxy-und Difluormethylthioarenen, welche als Zwischenprodukte zur Herstellung von biologisch aktiven Produkten verwendet werden können.

Es ist bekannt, daß man Difluormethoxyarene oder Difluormethylthioarene erhält, wenn man Hydroxyarene bzw. Mercaptoarene mit Chlordifluormethan in Gegenwart von Natriumhydroxid und unter Verwendung von Dioxan als Verdünnungsmittel umsetzt (vgl. J. Org. Chem. 25 (1960), 2009-2012; Zh. Obshch. Khim. 39 (1969), 206-210 - zitiert in Chem. Abstracts 70:96318d; Bull. Soc. Chim. Belg. 74 (1965), 270-280).

Mit dieser Synthesemethode werden jedoch zum Teil nur unbefriedigende Ausbeuten erzielt. Die Verwendung von Dioxan bringt zudem bei industrieller Anwendung Entsorgungsprobleme und die mit der Bildung von Peroxiden verbundenen Risiken mit sich.

Weiter ist bekannt, daß die Umsetzung von Hydroxyarenen mit Chlordifluormethan in Gegenwart von Natriumhydroxid auch unter Verwendung von Aceton/Wasser oder Aceton/Isopropanol/Wasser als Verdünnungsmitteln, gegebenenfalls in Gegenwart eines Phasentransfer-Katalysators, wie z.B. Benzyl-triethylammoniumchlorid, und gegebenenfalls unter erhöhtem Druck durchgeführt werden kann (vgl. EP-A 101570, US-P 4404148, US-P 4405529, US-P 4407760).

Auch bei dieser Verfahrensweise sind Ausbeute und Qualität der Produkte nicht ganz befriedigend.

Ferner ist bekannt, daß Difluormethoxyarene durch Umsetzung von Hydroxyarenen mit Chlordifluormethan in Gegenwart von Basen und unter Verwendung von hochpolaren, aprotischen Lösungsmitteln in guten Ausbeuten erhalten werden können (vgl. JP-A 59157041 - zitiert in Chem. Abstracts 102:78548d).

Die Verwendung von hochpolaren, aprotischen Lösungsmitteln sollte jedoch im industriellen Maßstab wegen der damit verbundenen ökologischen Probleme möglichst vermieden werden.

Weiterhin ist auch die Synthese von S-Alkylthio-benzolen (S-Alkylthiophenolen bzw. Phenyl-alkyl-thioethern) durch Alkylierung von Natrium-thiophenolat mit Alkylhalogeniden in Benzol/Wasser als Verdünnungsmittel und in Gegenwart von N,N'-Bis(dodecyldimethyl)-1,6-hexandiammonium-dibromid als Phasentransfer-Katalysator beschrieben worden (vgl. CS-A 266,277 - zitiert in Chem. Abstracts 115:279585f).

Bekannt ist ferner auch die Umsetzung von 1-(3-Nitrophenyl)-3-methyl-Δ²-1,2,4-triazolin-5-on mit Chlordifluormethan in Toluol/Wasser als Verdünnungsmittel sowie in Gegenwart von Natriumhydroxid und von Tetrabutylammoniumbromid als Phasentransfer-Katalysator, wobei unter N-Alkylierung das entsprechende 4-Difluormethyl-Derivat gebildet wird (vgl. JP-A 60 48975 - zitiert in Chem. Abstracts 103:104979n).

Schließlich ist bekannt, daß Difluormethoxy- bzw. Difluormethylthioarene auch durch Umsetzung entsprechender Hydroxy- bzw. Mercaptoarene mit Chlordifluormethan in mäßig polaren, aprotischen Lösungsmitteln unter Verwendung von festem Natriumhydroxid in Gegenwart von Tris-(3,6-dioxaheptyl)-amin erhalten werden können (vgl. J. Fluorine Chem. 41 (1988), 247-261).
Auch unter diesen Bedingungen werden die gewünschten Produkte oft in unbefriedigender Ausbeute und Qualität erhalten.

Des weiteren ist bekannt, daß Difluormethoxyphenyl-alkylketone durch Umsetzung von Hydroxyphenyl-alkylketonen in organischen Lösungsmitteln in Gegenwart einer Base und eines quartären Ammoniumsalzes als Phasentransferkatalysator hergestellt werden können (JP-A2-04 356 439, englische Übersetzung).

Es wurde nun gefunden, daß man Difluormethoxy- oder Difluormethylthioarene der allgemeinen Formel (I)

Ar-Q-CHF₂ (I)

in welcher
- Ar: für gegebenenfalls substituiertes Aryl steht und
- Q: für Sauerstoff oder Schwefel steht,
in sehr guten Ausbeuten und in hoher Reinheit erhält, wenn man
Hydroxy- oder Mercaptoarene der allgemeinen Formel (II)

Ar-Q-H (II)

in welcher
- Ar und Q: die oben angegebene Bedeutung haben,
mit Chlordifluormethan (ClCHF₂) in Gegenwart eines wäßrigen Alkalimetall- oder Erdalkalimetallhydroxids und eines tetrasubstituierten Phosphoniumsalzes als Phasentransfer-Katalysator bei Temperaturen zwischen 20°C und 120°C umsetzt, wobei die Umsetzung in Gegenwart eines unpolaren oder mäßig polaren Lösungsmittels aus der Reihe der aliphatischen oder alicyclischen Kohlenwasserstoffe oder der Reihe der gegebenenfalls durch Chlor substituierten aromatischen Kohlenwasserstoffe oder der Reihe der verzweigten Alkylether durchgeführt wird.

Überraschenderweise können nach dem erfindungsgemäßen Verfahren, bei welchem im Vergleich mit den bekannten Verfahren erheblich weniger Chlordifluormethan und erheblich weniger Alkalimetall- oder Erdalkalimetallhydroxid benötigt wird, die Verbindungen der Formel (I) in erheblich verbesserten Ausbeuten und praktisch frei von Nebenprodukten erhalten werden, wobei die Rückgewinnung der mit Wasser praktisch nicht mischbaren Lösungsmittel ohne großen Aufwand möglich ist.

Das erfindungsgemäße Verfahren stellt somit eine wertvolle Bereicherung des Standes der Technik dar.

Das erfindungsgemäße Verfahren betrifft vorzugsweise die Herstellung von Difluormethoxy- oder Difluormethylthioarenen der Formel (I), in welcher
- Ar: für jeweils gegebenenfalls substituiertes Phenyl oder Naphthyl steht, wobei die Substituenten vorzugsweise ausgewählt sind aus der Reihe Halogen, Nitro, Cyano, Carboxy, Carbamoyl, C₁-C₄-Alkyl (welches gegebenenfalls durch Halogen, Cyano, Carboxy, Carbamoyl, Hydroxy, Amino, C₁-C₄-Alkoxy, C₁-C₄-Alkoxy-carbonyl oder Phenyl oder durch geminale Bis-(C₁-C₄-alkoxy)-gruppen substituiert ist), C₁-C₄-Alkoxy (welches gegebenenfalls durch Halogen, Cyano, Carboxy, Carbamoyl, Hydroxy, Amino, C₁-C₄-Alkoxy oder C₁-C₄-Alkoxy-carbonyl substituiert ist), C₁-C₄-Alkylthio, C₁-C₄-Alkylsulfinyl oder C₁-C₄-Alkyl-sulfonyl (welche jeweils gegebenenfalls durch Halogen substituiert sind), C₁-C₄-Alkylamino, Di-(C₁-C₄-alkyl)-amino, C₁-C₄-Alkylcarbonyl, C₁-C₄-Alkoxy-carbonyl, C₁-C₄-Alkylamino-carbonyl, Di-(C₁-C₄-alkyl)-amino-carbonyl, C₁-C₄-Alkyl-carbonyloxy, C₁-C₄-Alkyl-carbonylamino, C₁-C₄-Alkoxy-carbonyloxy, C₁-C₄-Alkoxy-carbonylamino, C₁-C₄-Alkylamino-carbonyloxy oder C₁-C₄-Alkylamino-carbonylamino (welche jeweils gegebenenfalls durch Halogen oder C₁-C₄-Alkoxy substituiert sind), Phenyl, Phenoxy, Phenylthio, Phenylsulfinyl, Phenylsulfonyl, Phenylamino, Phenylcarbonyl, Phenylazo oder Phenylazoxy (welche jeweils gegebenenfalls Substituenten enthalten, wie sie oben beispielhaft angegeben sind), und
- Q: für Sauerstoff oder Schwefel steht.

Das erfindungsgemäße Verfahren betrifft insbesondere die Herstellung von Difluormethoxy- oder Difluormethylthioarenen der Formel (I), in welcher
- Ar: für jeweils gegebenenfalls substituiertes Phenyl oder Naphthyl steht, wobei die Substituenten vorzugsweise ausgewählt sind aus der Reihe
Fluor, Chlor, Brom, Nitro, Cyano, Carboxy, Carbamoyl, Methyl, Ethyl, n-oder i-Propyl (welche jeweils gegebenenfalls durch Fluor, Chlor, Cyano, Carboxy, Hydroxy, Methoxy, Ethoxy, Methoxycarbonyl, Ethoxycarbonyl oder Phenyl substituiert sind), Methoxy, Ethoxy, n- oder i-Propoxy (welche jeweils gegebenenfalls durch Fluor, Chlor, Cyano, Carboxy, Hydroxy, Methoxy, Ethoxy, Methoxycarbonyl oder Ethoxycarbonyl substituiert sind), Methylthio, Ethylthio, Methylsulfinyl, Ethylsulfinyl, Methylsulfonyl oder Ethylsulfonyl (welche jeweils gegebenenfalls durch Fluor oder Chlor substituiert sind), Methylamino, Ethylamino, Dimethylamino, Diethylamino, Acetyl, Propionyl, Methoxycarbonyl, Ethoxycarbonyl, Methylaminocarbonyl, Ethylaminocarbonyl, Dimethylaminocarbonyl, Diethylaminocarbonyl, Acetyloxy, Propionyloxy, Acetylamino, Propionylamino, Methoxycarbonyloxy, Ethoxycarbonyloxy, Methoxycarbonylamino, Ethoxycarbonylamino, Methylaminocarbonyloxy, Ethylaminocarbonyloxy, Methylaminocarbonylamino, Ethylaminocarbonylamino (welche jeweils gegebenenfalls durch Fluor, Chlor, Methoxy oder Ethoxy substituiert sind), Phenyl, Phenoxy, Phenylthio, Phenylsulfinyl, Phenylsulfonyl, Phenylamino oder Phenylcarbonyl (welche jeweils gegebenenfalls Substituenten enthalten, wie sie oben beispielhaft angegeben sind), und
- Q: für Sauerstoff oder Schwefel steht.

Im Falle, daß in Formel (I) der Rest Ar durch Alkoxy substituiertes Alkyl enthält, sind in die Definitionen ausdrücklich auch geminale Bis-Alkoxy-alkylreste als Substituenten eingeschlossen (d.h. Aldehyd- und Keton-Acetale).

Als Beispiele für die Verbindungen, welche nach dem erfindungsgemäßen Verfahren besonders gut hergestellt werden können, seien genannt:
1-Difluormethoxy-2-nitro-benzol, 1-Difluormethoxy-3-nitro-benzol, 1-Difluormethoxy-4-nitro-benzol und 4-Difluormethoxy-acetophenon.

Verwendet man beispielsweise 2-Fluor-phenol und Chlordifluormethan als Ausgangsstoffe, so kann der Reaktionsablauf beim erfindungsgemäßen Verfahren durch das folgende Formelschema skizziert werden:

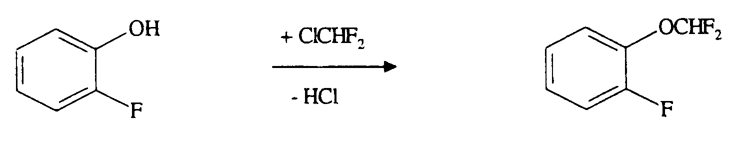

Die beim erfindungsgemäßen Verfahren als Ausgangsstoffe zu verwendenden Hydroxy- oder Mercaptoarene sind durch die Formel (II) allgemein definiert. In der Formel (II) haben Ar und Q vorzugsweise bzw. insbesondere diejenigen Bedeutungen, die bereits oben im Zusammenhang mit der Beschreibung der erfindungsgemäß herzustellenden Verbindungen der Formel (I) vorzugsweise bzw. als insbesondere bevorzugt für Ar und Q angegeben wurden.

Als Beispiele für die Ausgangsstoffe der Formel (II) seien genannt:
2-Nitro-phenol, 3-Nitro-phenol, 4-Nitro-phenol und 4-Hydroxy-acetophenon.

Die Ausgangsstoffe der Formel (II) sind - wie auch das weiter als Ausgangsverbindung benötigte Chlordifluormethan - bekannte organische Chemikalien.

Das erfindungsgemäße Verfahren wird unter Verwendung eines Alkalimetall- oder Erdalkalimetallhydroxids durchgeführt.

Als Beispiele für geeignete Alkalimetall- und Erdalkalimetallhydroxide seien genannt: Lithiumhydroxid, Natriumhydroxid, Kaliumhydroxid, Magnesiumhydroxid und Calcium-hydroxid.
Besonders bevorzugt wird Natriumhydroxid.

Das erfindungsgemäße Verfahren wird unter Verwendung eines Phasentransfer-Katalysators aus der Reihe der tetrasubstituierten Phosphonium-salze (-halogenide, -hydroxide, -hydrogensulfate, -tetrafluoroborate etc.) durchgeführt.

Als Beispiele für solche Katalysatoren seien genannt: Tetrabutylphosphoniumbromid, Tetrabutylphosphoniumchlorid, Tributyl-hexadecylphosphoniumbromid, Butyl-triphenylphosphoniumchlorid, Ethyl-trioctylphosphoniumbromid, Tetraphenylphosphoniumbromid. Diese Phasentransfer-Katalysatoren sind handelsübliche Chemikalien.

Das erfindungsgemäße Verfahren wird unter Verwendung eines unpolaren oder mäßig polaren Lösungsmittels aus der Reihe der aliphatischen oder alicyclischen Kohlenwasserstoffe oder der Reihe der gegebenenfalls durch Chlor substituierten aromatischen Kohlenwasserstoffe oder der Reihe der verzweigten Alkylether durchgeführt.

Als Beispiele für diese Lösungsmittel seien genannt:
Pentan, Hexan, Heptan, Octan (jeweils geradkettig oder verzweigt), Cyclohexan, Methylcyclohexan, Petrolether, Benzin, Ligroin, Benzol, Toluol, Xylol (alle Isomeren), Cumol, Chlorbenzol, o-Dichlorbenzol, Diisopropylether, Diisobutylether, Methyl-sec-butylether, Methyl-tert-butylether, Methyl-sec-pentylether, Methyl-tert-pentylether.

Besonders bevorzugt werden Toluol, Xylol (alle Isomeren), Methyl-tert-butylether und Methyl-tert-pentylether.

Die Reaktionstemperaturen können bei der Durchführung des erfindungsgemäßen Verfahrens in einem größeren Bereich variiert werden. Im allgemeinen arbeitet man bei Temperaturen zwischen 20°C und 120°C, vorzugsweise zwischen 40°C und 110°C, insbesondere zwischen 60°C und 100°C.

Zur Durchführung des erfindungsgemäßen Verfahrens setzt man auf 1 Mol Ausgangsverbindung der Formel (II) im allgemeinen zwischen 1 und 5 Mol, vorzugsweise zwischen 1,2 und 4,5 Mol Chlordifluormethan, zwischen 1,5 und 2,5 Moläquivalenten, vorzugsweise zwischen 1,8 und 2,2 Moläquivalenten Alkalimetall- oder Erdalkalimetallhydroxid und zwischen 1 Mol% und 10 Mol%, vorzugsweise zwischen 2 Mol% und 5 Mol% Phasentransfer-Katalysator ein.

In einer bevorzugten Ausführungsform des erfindungsgemäßen Verfahrens werden die Ausgangsverbindung der Formel (II), das in Wasser gelöste oder dispergierte Alkalimetall-oder Erdalkalimetallhydroxid, das unpolare oder mäßig polare Lösungsmittel und der Phasentransfer-Katalysator vermischt und in diese Mischung wird unter starkem Rühren bei der geeigneten Umsetzungstemperatur Chlordifluormethan eingeleitet, bis das Ende der Umsetzung - erkennbar z.B. am Farbumschlag - erreicht ist.

Die Aufarbeitung und Isolierung der Produkte der Formel (I) kann nach üblichen Methoden durchgeführt werden. Beispielsweise wird der Ansatz mit Wasser verdünnt, die organische Phase abgetrennt und die wäßrige Phase mit dem jeweils verwendeten organischen Lösungsmittel nachextrahiert. Die vereinigten organischen Phasen werden mit Wasser gewaschen, mit Natriumsulfat getrocknet und filtriert. Das Filtrat wird dann im Wasserstrahlvakuum eingeengt und das als Rückstand erhaltene Rohprodukt nach üblichen Methoden (z.B. durch Vakuumdestillation) gereinigt (vergleiche auch die Herstellungsbeispiele).

Die nach dem erfindungsgemäßen Verfahren herstellbaren Verbindungen können als Zwischenprodukte zur Herstellung von biologisch aktiven Produkten, beispielsweise von Herbiziden (vgl. DE-OS 3503773 = EP-A-192 998 = US 4.769.062; ferner EP-A-23 422 = US 4.452.628; US 4.534.788) oder von Insektiziden (vgl. EP-A 58424, US-P 4464386, EP-A 277091, EP-A 466408, US-P 5109014, EP-A 490569) verwendet werden.

Nach dem erfindungsgemäßen Verfahren lassen sich auch 1-Difluormethoxy-2-nitrobenzol und 1-Difluormethoxy-4-nitrobenzol durch Umsetzung von 2-Nitrophenol beziehungsweise 4-Nitrophenol jeweils mit Chlordifluormethan in Gegenwart eines Phasentransfer-Katalysators aus der Reihe der tetrasubstituierten Ammoniumsalze in hohen Ausbeuten herstellen.

Dieses erfindungsgemäße Verfahren zur Herstellung von 1-Difluormethoxy-2-nitrobenzol und 1-Difluormethoxy-4-nitrobenzol wird auch unter Verwendung eines Phasentransfer-Katalysators aus der Reihe der tetrasubstituierten Ammonium-salze (-halogenide, -hydroxide, -hydrogensulfate, tetrafluoroborate) durchgeführt.

Als Beispiel für solche Katalysatoren seien genannt:
Tetrabutylammoniumbromid, Tetrabutylammoniumchlorid, Tetraoctylammoniumchlorid, Tetrabutylammoniumhydrogensulfat, Methyl-trioctylammoniumchlorid, Hexadecyl-trimethylammoniumchlorid, Hexadecyl-trimethylammoniumbromid, Benzyltrimethyl-ammoniumchlorid, Benzyl-triethylammoniumchlorid, Benzyl-trimethylammoniumhydroxid, Benzyl-triethylammoniumhyroxid, Benzyl-tributylammoniumchlorid und Benzyl-tributylammoniumbromid.

### Herstellungsbeispiele:

### Beispiel 1

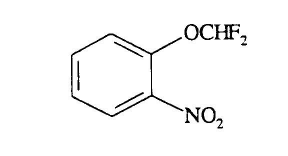

In eine intensiv gerührte Mischung aus 139 g (1,0 Mol) 2-Nitro-phenol, 80 g (2,0 Mol) Natriumhydroxid, 120 ml Wasser, 1400 ml Toluol und 16 g (0,05 Mol) Tetrabutylammoniumbromid werden bei 90°C 322 g (3,72 Mol) Chlordifluormethan eingeleitet.

Wenn die Farbe der Reaktionsmischung von rot nach hellgelb umgeschlagen ist, läßt man auf ca. 20°C abkühlen, verdünnt mit Wasser auf etwa das doppelte Volumen und trennt die organische Phase ab. Die wäßrige Phase wird noch zweimal mit Toluol nachextrahiert. Dann werden die vereinigten organischen Phasen mit Wasser gewaschen, mit Natriumsulfat getrocknet und filtriert. Das Filtrat wird im Wasserstrahlvakuum eingeengt und das als Rückstand verbliebene Rohprodukt durch Vakuumdestillation gereinigt.

Man erhält 177 g (94% der Theorie) 1-Difluormethoxy-2-nitrobenzol vom Siedebereich 60°C - 62°C (bei 0,3 mbar). (Gehalt nach HPLC: 99,95%).
(vgl. Zh. Obshch. Khim. 39 (1969), 206-210; Ausbeute: 80% der Theorie).

### Beispiel 2

Bei analoger Durchführung wie in Beispiel 1, wobei lediglich Xylol statt Toluol als Lösungsmittel verwendet wird und nur 123 g (1,42 Mol) Chlordifluormethan verbraucht werden, erhält man 170 g (Gehalt: 98,3% , d.h. 88,4% der Theorie) 1-Difluormethoxy-2-nitro-benzol.

### Beispiel 3

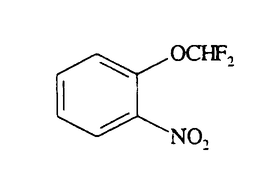

Eine Mischung aus 14,0 g (0,1 Mol) 2-Nitro-phenol, 8,0 g (0,2 Mol) Natriumhydroxid, 12 ml Wasser, 140 ml Methyl-tert-pentylether und 1,6 g (0,005 Mol) Tetrabutylammoniumbromid wird mit 39 g (0,45 Mol) Chlordifluormethan wie in Beispiel 1 beschrieben umgesetzt und ebenso aufgearbeitet.

Man erhält 17,4 g (Gehalt: 99,2%; d.h. 91,3% der Theorie) 1-Difluormethoxy-2-nitro-benzol.

### Beispiel 4

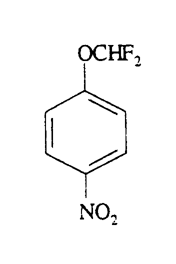

141,7 g (Gehalt: 98%; 1,0 Mol) 4-Nitro-phenol werden in 1400 ml Toluol gelöst und unter Rühren wird vorsichtig eine 40%ige wäßrige Natriumhydroxid-Lösung zugetropft. Nach Erwärmen der Mischung auf 90°C werden 17 g (0,05 Mol) Tetrabutylphosphoniumbromid dazugegeben und dann werden unter Rühren 150 g (1,73 Mol) Chlordifluormethan eingeleitet. Anschließend wird wie unter Beispiel 1 beschrieben aufgearbeitet.

Man erhält 183,1 g (Gehalt: 100%; 96,8% der Theorie) 1-Difluormethoxy-4-nitrobenzol vom Siedepunkt 120°C (bei 18,6 mbar), welches allmählich kristallisiert (Schmelzpunkt: 35°C).
(vgl. JP-A59157041: Ausbeute: 77% der Theorie)

## Patentansprüche

1. Verfahren zur Herstellung von Difluormethoxy- und Difluormethylthioarenen der allgemeinen Formel (I),
Ar-Q-CHF₂ (I)
in welcher
Ar für gegebenenfalls substituiertes Aryl steht und
Q für Sauerstoff oder Schwefel steht,
durch Umsetzung von Hydroxy- oder Mercaptoarenen der allgemeinen Formel (II)
Ar-Q-H (II
in welcher
Ar und Q die oben angegebene Bedeutung haben,
mit Chlordifluormethan (ClCHF₂) in Gegenwart eines wäßrigen Alkalimetall- oder Erdalkalimetallhydroxids und in Gegenwart eines Phasentransfer-Katalysators bei Temperaturen zwischen 20°C und 120°C, dadurch gekennzeichnet, daß als Phasentransfer-Katalysator ein tetrasubstituiertes Phosphoniumsalz verwendet wird und daß die Umsetzung in Gegenwart eines unpolaren oder mäßig polaren Lösungsmittels aus der Reihe der aliphatischen oder alicyclischen Kohlenwasserstoffe oder der Reihe der gegebenenfalls durch Chlor substituierten aromatischen Kohlenwasserstoffe oder der Reihe der verzweigten Alkylether durchgeführt wird.

2. Verfahren nach Anspruch 1, dadurch gekennzeichnet, daß man bei Temperaturen zwischen 40°C und 110°C arbeitet.

3. Verfahren nach Anspruch 1, dadurch gekennzeichnet, daß man bei Temperaturen zwischen 60°C und 100°C arbeitet.

4. Verfahren nach Anspruch 1, dadurch gekennzeichnet, daß man auf 1 Mol Ausgangsverbindung der Formel (II) zwischen 1 und 5 Mol (vorzugsweise zwischen 1,2 und 4,5 Mol) Chlordifluormethan (ClCHF₂), zwischen 1,5 und 2,5 Moläquivalenten (vorzugsweise zwischen 1,8 und 2,2 Moläquivalenten) Alkalimetall- oder Erdalkalimetallhydroxid und zwischen 1 Mol-% und 10 Mol-% (vorzugsweise zwischen 2 Mol-% und 5 Mol-%) Phasentransfer-Katalysator einsetzt.

5. Verfahren nach Anspruch 1, dadurch gekennzeichnet, daß man als Phasentransfer-Katalysator eine der nachfolgend genannten Phosphonium-Verbindungen einsetzt:
Tetrabutylphosphoniumbromid, Tetrabutylphosphoniumchlorid, Tributyl-hexadecylphosphoniumbromid, Butyl-triphenylphosphoniumchlorid, Ethyl-trioctylphosphoniumbromid oder Tetraphenylphosphoniumbromid.

6. Verfahren nach Anspruch 1, dadurch gekennzeichnet, daß man als unpolares oder mäßig polares Lösungsmittel eines der nachfolgend genannten Lösungsmittel einsetzt:
Pentan, Hexan, Heptan, Octan (jeweils geradkettig oder verzweigt), Cyclohexan, Methylcyclohexan, Petrolether, Benzin, Ligroin, Benzol, Toluol, Xylol (alle Isomeren), Cumol, Chlorbenzol, o-Dichlorbenzol, Diisopropylether, Diisobutylether, Methyl-sec-butylether, Methyl-tert-butylether, Methyl-sec-pentylether oder Methyl-tert-pentylether.

7. Verfahren nach Anspruch 1, dadurch gekennzeichnet, daß man als Alkalimetallhydroxid Natriumhydroxid einsetzt.

8. Verfahren nach Anspruch 1, dadurch gekennzeichnet, daß man die Ausgangsverbindung der Formel (II), das in Wasser gelöste oder dispergierte Alkalimetall- oder Erdalkalimetallhydroxid, das unpolare oder mäßig polare Lösungsmittel und den Phasentransfer-Katalysator vermischt und in diese Mischung unter starkem Rühren bei der geeigneten Umsetzungstemperatur Chlordifluormethan einleitet, bis das Ende der Umsetzung erreicht ist.

9. Verfahren zur Herstellung von 1-Difluormethoxy-4-nitro-benzol durch Umsetzung von 4-Nitro-phenol mit Chlordifluormethan (ClCHF₂) gemäß Anspruch 1.

10. Verfahren zur Herstellung von 1-Difluormethoxy-2-nitro-benzol und 1-Difluormethoxy-4-nitro-benzol durch Umsetzung von 2-Nitro-phenol beziehungsweise von 4-Nitro-phenol jeweils mit Chlordifluormethan (ClCHF₂) in Gegenwart eines wäßrigen Alkalimetall- oder Erdalkalimetallhydroxids und in Gegenwart eines Phasentransfer-Katalysators aus der Reihe der tetrasubstituierten Ammoniumsalze bei Temperaturen zwischen 20°C und 120°C, dadurch gekennzeichnet, daß die Umsetzung in Gegenwart eines unpolaren oder mäßig polaren Lösungsmittels aus der Reihe der aliphatischen oder alicyclischen Kohlenwasserstoffe oder der Reihe der gegebenenfalls durch Chlor substituierten aromatischen Kohlenwasserstoffe oder der Reihe der verzweigten Alkylether durchgeführt wird.

## Claims

1. Process for preparing difluoromethoxyarenes and difluoromethylthioarenes of the general formula (I),
Ar-Q-CHF₂ (I)
in which
Ar represents optionally substituted aryl, and
Q represents oxygen or sulphur,
by reacting hydroxyarenes or mercaptoarenes of the general formula (II)
Ar-Q-H (II)
in which
Ar and Q have the abovementioned meaning,
with chlorodifluoromethane (ClCHF₂) in the presence of an aqueous alkali metal hydroxide or alkaline earth metal hydroxide and in the presence of a phase-transfer catalyst at temperatures of between 20°C and 120°C, characterized in that a tetrasubstituted phosphonium salt is used as the phase-transfer catalyst, and in that the reaction is carried out in the presence of a non-polar or moderately polar solvent from the group comprising the aliphatic or alicyclic hydrocarbons or the group comprising the aromatic hydrocarbons which are optionally substituted by chlorine or the group comprising the branched alkyl ethers.

2. Process according to Claim 1, characterized in that temperatures of between 40°C and 110°C are employed.

3. Process according to Claim 1, characterized in that temperatures of between 60°C and 100°C are employed.

4. Process according to Claim 1, characterized in that between 1 and 5 mol (preferably between 1.2 and 4.5 mol) of chlorodifluoromethane (ClCHF₂), between 1.5 and 2.5 molar equivalents (preferably between 1.8 and 2.2 molar equivalents) of alkali metal hydroxide or alkaline earth metal hydroxide, and between 1 mol% and 10 mol% (preferably between 2 mol% and 5 mol%) of phase-transfer catalyst are employed per 1 mol of starting compound of the formula (II).

5. Process according to Claim 1, characterized in that one of the phosphonium compounds named below is employed as the phase-transfer catalyst:
tetrabutylphosphonium bromide, tetrabutylphosphonium chloride, tributyl-hexadecylphosphonium bromide, butyl-triphenylphosphonium chloride, ethyl-trioctylphosphonium bromide or tetraphenylphosphonium bromide.

6. Process according to Claim 1, characterized in that one of the solvents named below is employed as the non-polar or moderately polar solvent:
pentane, hexane, heptane, octane (in each case straight-chain or branched), cyclohexane, methylcyclohexane, petroleum ether, benzine, ligroin, benzene, toluene, xylene (all the isomers), cumene, chlorobenzene, o-dichlorobenzene, diisopropyl ether, diisobutyl ether, methyl sec-butyl ether, methyl tert-butyl ether, methyl sec-pentyl ether and methyl tert-pentyl ether.

7. Process according to Claim 1, characterized in that sodium hydroxide is employed as the alkali metal hydroxide.

8. Process according to Claim 1, characterized in that the starting compound of the formula (II), the alkali metal hydroxide or alkaline earth metal hydroxide which is dissolved or dispersed in water, the non-polar or moderately polar solvent and the phase-transfer catalyst are mixed together and chlorodifluoromethane is then passed into this mixture, at the appropriate reaction temperature and while stirring vigorously, until the reaction is complete.

9. Process for preparing 1-difluoromethoxy-4-nitro-benzene by reacting 4-nitro-phenol with chlorodifluoromethane (ClCHF₂) in accordance with Claim 1.

10. Process for preparing 1-difluoromethoxy-2-nitro-benzene and 1-difluoromethoxy-4-nitro-benzene by reacting 2-nitro-phenol and 4-nitro-phenol, respectively, in each case with chlorodifluoromethane (ClCHF₂) in the presence of an aqueous alkali metal hydroxide or alkaline earth metal hydroxide and in the presence of a phase-transfer catalyst from the group comprising the tetrasubstituted ammonium salts at temperatures of between 20°C and 120°C, characterized in that the reaction is carried out in the presence of a non-polar or moderately polar solvent from the group comprising the aliphatic or alicyclic hydrocarbons or the group comprising the aromatic hydrocarbons which are optionally substituted by chlorine or the group comprising the branched alkyl ethers.

## Revendications

1. Procédé pour la préparation de difluorométhoxy- et de difluorométhylthioarènes répondant à la formule générale (I)
Ar-Q-CHF₂ (I)
dans laquelle
Ar représente un groupe aryle éventuellement substitué, et
Q représente un atome d'oxygène ou un atome de soufre,
par mise en réaction d'hydroxy- ou de mercaptoarènes répondant à la formule générale (II)
Ar-Q-H (II)
dans laquelle Ar et Q ont la signification indiquée ci-dessus,
avec du chlorodifluorométhane (ClCHF₂) en présence d'un hydroxyde aqueux de métal alcalin ou de métal alcalino-terreux et en présence d'un catalyseur de transfert de phase, à des températures entre 20°C et 120°C, caractérisé en ce qu'on utilise, à titre de catalyseur de transfert de phase, un sel de phosphonium tétrasubstitué et en ce qu'on effectue la mise en réaction en présence d'un solvant apolaire ou modérément polaire choisi parmi la série des hydrocarbures aliphatiques ou alicycliques ou parmi la série des hydrocarbures aromatiques portant éventuellement un ou plusieurs substituants chloro, ou encore parmi la série des éthers alkyliques ramifiés.

2. Procédé selon la revendication 1, caractérisé en ce qu'on travaille à des températures entre 40°C et 110 °C.

3. Procédé selon la revendication 1, caractérisé en ce qu'on travaille à des températures entre 60°C et 100°C.

4. Procédé selon la revendication 1, caractérisé en ce qu'on met en oeuvre, pour 1 mole du composé de départ de formule (II), entre 1 et 5 moles (de préférence entre 1,2 et 4,5 moles) de chlorodifluorométhane (ClCHF₂), entre 1,5 et 2,5 équivalents molaires (de préférence entre 1,8 et 2,2 équivalents molaires) d'hydroxyde de métal alcalin ou de métal alcalino-terreux et entre 1 mole % et 10 moles % (de préférence entre 2 moles % et 5 moles %) de catalyseur de transfert de phase.

5. Procédé selon la revendication 1, caractérisé en ce qu'on met en oeuvre, comme catalyseur de transfert de phase, un des composés de phosphonium mentionnés ci-après:
le bromure de tétrabutyl-phosphonium, le chlorure de tétrabutyl-phosphonium, le bromure de tributyl-hexadécyl-phosphonium, le chlorure de butyl-triphényl-phosphonium, le bromure d' éthyl-trioctyl-phosphonium ou le bromure de tétraphényl-phosphonium.

6. Procédé selon la revendication 1, caractérisé en ce qu'on met en oeuvre, comme solvant apolaire ou modérément polaire, un des solvants mentionnés ci-après:
le pentane, l'hexane, l'heptane, l'octane (respectivement à chaîne droite ou ramifiée), le cyclohexane, le méthyl-cyclohexane, l'éther de pétrole, l'essence, la ligroïne, le benzène, le toluène, le xylène (tous les isomères), le cumène, le chlorobenzène, le o-dichlorobenzène, l'éther diisopropylique, l'éther diisobutylique, l'éther méthyl-sec.-butylique, l'éther méthyl-tert.-butylique, l'éther méthyl-sec.-pentylique ou l'éther méthyl-tert.-pentylique.

7. Procédé selon la revendication 1, caractérisé en ce qu'on met en oeuvre, comme hydroxyde de métal alcalin, l'hydroxyde de sodium.

8. Procédé selon la revendication 1, caractérisé en ce qu'on mélange le composé de départ de formule (II), l'hydroxyde de métal alcalin ou de métal alcalino-terreux dissous ou dispersé dans l'eau, le solvant apolaire ou modérément polaire et le catalyseur de transfert de phase et, tout en agitant vigoureusement, à la température de mise en réaction appropriée, on introduit dans ce mélange du chlorodifluorométhane jusqu'à ce que la mise en réaction arrive à son terme.

9. Procédé pour la préparation du 1-difluorométhoxy-4-nitrobenzène par mise en réaction du 4-nitrophénol avec du chlorodifluorométhane (ClCHF₂) selon la revendication 1.

10. Procédé pour la préparation du 1-difluorométhoxy-2-nitrobenzène et du 1-difluorométhoxy-4-nitrobenzène par mise en réaction du 2-nitrophénol, respectivement du 4-nitrophénol chaque fois avec du chlorodifluorométhane (ClCHF₂) en présence d'un hydroxyde aqueux de métal alcalin ou de métal alcalino-terreux et en présence d'un catalyseur de transfert de phase choisi parmi la série des sels d'ammonium tétrasubstitués, à des températures entre 20°C et 120°C, caractérisé en ce qu'on effectue la mise en réaction en présence d'un solvant apolaire ou modérément polaire choisi parmi la série des hydrocarbures aliphatiques ou alicycliques ou parmi la série des hydrocarbures aromatiques portant éventuellement un ou plusieurs substituants chloro, ou encore parmi la série des éthers alkyliques ramifiés.
